Europäisches Patentamt

European Patent Office

Office européen des brevets

(19)

(11) **EP 0 597 210 B1**

(12) **EUROPÄISCHE PATENTSCHRIFT**

(45) Veröffentlichungstag und Bekanntmachung des Hinweises auf die Patenterteilung:
13.11.1996 Patentblatt 1996/46

(51) Int. Cl.$^6$: **C07C 31/30**, C07C 29/68

(21) Anmeldenummer: 93114903.3

(22) Anmeldetag: 16.09.1993

(54) **Lagerstabile Lösungen von carbonisiertem Magnesiumethylat in Ethanol sowie deren Herstellung und Verwendung**

Storage stable solutions of carbonated magnesium ethoxid in ethanol, their use and production

Solutions stable au stockage d'éthylate de magnésium carbonatisé dans l'alcool éthylique ainsi que leur préparation et leur utilisation

(84) Benannte Vertragsstaaten:
AT BE DE ES FR GB IT NL

(30) Priorität: 07.11.1992 DE 4237701

(43) Veröffentlichungstag der Anmeldung:
18.05.1994 Patentblatt 1994/20

(73) Patentinhaber: HÜLS AKTIENGESELLSCHAFT
45764 Marl (DE)

(72) Erfinder:
• **Rauleder, Hartwig, Dr.**
**D-79618 Rheinfelden (DE)**
• **Standke, Burkhard, Dr.**
**D-79618 Rheinfelden (DE)**
• **Kötzsch, Hans-Joachim, Dr.**
**D-79618 Rheinfelden (DE)**
• **Schork, Reinhold, Dr.**
**D-79618 Rheinfelden (DE)**

(56) Entgegenhaltungen:
EP-A- 0 236 082          EP-A- 0 491 128
US-A- 3 277 002

## Beschreibung

Die Erfindung betrifft lagerstabile Lösungen von carbonisiertem Magnesiumethylat der Formel $Mg(C_2H_5O)_2 (CO_2)_n$ in Ethanol.

Des weiteren betrifft die Erfindung ein Verfahren zur Herstellung lagerstabiler Lösungen von carbonisiertem Magnesiumethylat der Formel $Mg(C_2H_5O)_2 (CO_2)_n$ in Ethanol durch Umsetzung von metallischem Magnesium mit Ethanol und $CO_2$ sowie ein Verfahren zur Herstellung lagerstabiler Lösungen von carbonisiertem Magnesiumethylat der Formel $Mg(C_2H_5O)_2 (CO_2)_n$ in Ethanol durch Umsetzung von Magnesiumethylat in Ethanol mit $CO_2$.

Außerdem betrifft die Erfindung die Verwendung der lagerstabilen Lösungen von carbonisiertem Magnesiumethylat in Ethanol zur Konservierung von Papier sowie die Verwendung zur Herstellung von Katalysatoren für die Polymerisierung von Olefinen.

Magnesiumalkoholate sind in der Regel in den korrespondierenden Alkoholen wenig löslich bzw. nahezu unlöslich. Eine Ausnahme bildet Magnesiummethylat, das in Methanol eine Löslichkeit bis zu ca. 12 Gew.-% aufweist.

Eine technisch einfache Methode, die Löslichkeit von Magnesiumalkoholaten zu erhöhen, stellt die Carbonisierung dar. In eine Suspension des Magnesiumalkoholates im korrespondierenden Alkohol wird gasförmiges, flüssiges oder festes $CO_2$ eingebracht. Hierbei bildet sich ein lösliches $CO_2$-Addukt. Das $CO_2$-Addukt ist im Falle von Magnesiumethylat in Ethanol mit einer Konzentration von über 30 Gew.-% löslich, hingegen ist reines Magnesiumethylat in Ethanol nahezu unlöslich.

Alkohollösliche carbonisierte Magnesiumalkoxide finden vielfache Anwendung. So können beispielsweise durch Carbonisierung in Lösung gebrachte Magnesiumalkoxide bei der Langzeitkonservierung von Papier, insbesondere von Büchern, verwendet werden und die zu diesem Zweck ebenfalls eingesetzten, in der Anwendung jedoch problematischen, Zinkalkyle substituieren.

Ein weiteres Anwendungsfeld ist die Herstellung von Katalysatoren für die Polymerisierung von Olefinen. Hierfür benötigt man z. B. sphärisches Magnesiumethylat, das beispielsweise durch Sprühtrocknung oder homogene Fällung carbonisierter Magnesiumethylatlösungen und gegebenenfalls anschließende Decarbonisierung hergestellt werden kann.

Es ist prinzipiell bekannt, daß Lösungen carbonisierter Magnesiumalkoholate nach den beiden folgenden Reaktionsschemata hergestellt werden können, wobei einmal metallisches Magnesium mit einem Alkohol ROH und $CO_2$ (siehe Reaktionsgleichung 1) und zum anderen ein Magnesiumalkoxid mit $CO_2$ (siehe Reaktionsgleichung 2) umgesetzt werden:

$$Mg + 2\,ROH + n\,CO_2 \rightarrow Mg(OR)_2 (CO_2)_n + H_2 \quad (1)$$

$$Mg(OR)_2 + n\,CO_2 \rightarrow Mg(OR)_2 (CO_2)_n \quad (2)$$

Hierbei bedeutet R eine Alkylgruppe, und für n gilt: $0 \leq n \leq 2$.

So lehrt EP-PS 0 236 082 die Herstellung von carbonisierten Magnesiumalkoxiden durch Reaktion von Magnesiumalkoxiden mit $CO_2$ in einem Lösungsmittel. Bevorzugte Lösungsmittel sind Alkohole. Beispielsweise wird auch die Herstellung von carbonisiertem Magnesiumethylat durch Umsetzung von Magnesiumethylat mit $CO_2$ in Ethanol als Lösungsmittel offenbart.

Fieser und Fieser beschreiben in "Reagents for Organic Synthesis", Vol. 1, Seite 631, die Herstellung von carbonisiertem Magnesiummethylat auf zwei verschiedenen Wegen:
Zum einen wird eine Suspension von Magnesiummethylat in Methanol mit $CO_2$ umgesetzt.
Zum anderen läßt man Magnesiumspäne mit Methanol zu Magnesiummethylat reagieren. Nachdem der Methanol teilweise bei 50 °C und Unterdruck abgezogen worden ist, wird Dimethylformamid als Lösungsmittel zugesetzt und $CO_2$ in diese Lösung eingeleitet. Der restliche Methanol wird abdestilliert, und man erhält eine schwach gelbe Lösung von carbonisiertem Magnesiummethylat in Dimethylformamid.

Die alkoholischen Lösungen carbonisierter Magnesiumalkoxide gemäß dem Stand der Technik sind jedoch stark gelb bis rot gefärbt, wobei sich die Verfärbung mit zunehmender Lagerdauer verstärken kann. Hinzu kommt, daß bei Lagerung über mehrere Wochen Ausfällungen oder Gelation auftreten können. Die technische Verwendung solcher Lösungen ist erheblich eingeschränkt: Einerseits können verfärbte Lösungen beispielsweise nicht für die Langzeitkonservierung von Büchern verwendet werden, andererseits sind Lösungen, bei denen die Gefahr unkontrollierter Ausfällungen besteht, für die Herstellung von Katalysatoren auf Basis Magnesiumalkoxid unbrauchbar.

Der Erfindung liegt die Aufgabe zugrunde, lagerstabile Lösungen von carbonisiertem Magnesiumethylat herzustellen, die auch über einen längeren Zeitraum nahezu farblos bleiben und weder Ausfällungen noch Gelierung zeigen.

Es wurde nun überraschenderweise gefunden, daß durch Einstellung definierter Magnesium- und $CO_2$-Konzentrationen in den erfindungsgemäßen Lösungen von carbonisiertem Magnesiumethylat Verfärbungen, Ausfällungen und Gelierungen auch während der Lagerung über lange Zeiträume nicht auftreten.

Gegenstand der vorliegenden Erfindung sind daher lagerstabile Lösungen von carbonisiertem Magnesiumethylat der Formel $Mg(C_2H_5O)_2 (CO_2)_n$ in Ethanol, die dadurch gekennzeichnet sind, daß der Magnesiumgehalt der Lösung 2,5 bis 6 Gew.-%, bezogen auf die gesamte Lösung, und der $CO_2$-Gehalt n 1,55 bis 1,85 beträgt

oder der Magnesiumgehalt der Lösung 1,5 bis 2,5 Gew.-%, bezogen auf die gesamte Lösung, und der $CO_2$-Gehalt n 1,55 bis 1,90 beträgt

oder der Magnesiumgehalt der Lösung kleiner als 1,5 Gew.-%, bezogen auf die gesamte Lösung, ist und der $CO_2$-Gehalt n 1,55 bis 2,2 beträgt.

Des weiteren ist Gegenstand der vorliegenden Erfindung ein Verfahren zur Herstellung lagerstabiler Lösungen von carbonisiertem Magnesiumethylat der Formel $Mg(C_2H_5O)_2 (CO_2)_n$ in Ethanol durch Umsetzung von metallischem Magnesium mit Ethanol und $CO_2$, das dadurch gekennzeichnet ist,
daß der Magnesiumgehalt der Lösung auf Werte von 2,5 bis 6 Gew.-%, bezogen auf die gesamte Lösung, und der $CO_2$-Gehalt auf Werte von n gleich 1,55 bis 1,85
oder der Magnesiumgehalt der Lösung auf Werte von 1,5 bis 2,5 Gew.-%, bezogen auf die gesamte Lösung, und der $CO_2$-Gehalt auf Werte von n gleich 1,55 bis 1,90
oder der Magnesiumgehalt der Lösung auf Werte von kleiner als 1,5 Gew.-%, bezogen auf die gesamte Lösung, und der $CO_2$-Gehalt auf Werte von n gleich 1,55 bis 2,2 eingestellt werden sowie
ein Verfahren zur Herstellung lagerstabiler Lösungen von carbonisiertem Magnesiumethylat der Formel $Mg(C_2H_5O)_2 (CO_2)_n$ in Ethanol durch Umsetzung von Magnesiumethylat in Ethanol mit $CO_2$, das dadurch gekennzeichnet ist,
daß der Magnesiumgehalt der Lösung auf Werte von 2,5 bis 6 Gew.-%, bezogen auf die gesamte Lösung, und der $CO_2$-Gehalt auf Werte von n gleich 1,55 bis 1,85
oder der Magnesiumgehalt der Lösung auf Werte von 1,5 bis 2,5 Gew.-%, bezogen auf die gesamte Lösung, und der $CO_2$-Gehalt auf Werte von n gleich 1,55 bis 1,90
oder der Magnesiumgehalt der Lösung auf Werte von kleiner als 1,5 Gew.-%,
bezogen auf die gesamte Lösung, und der $CO_2$-Gehalt auf Werte von n gleich 1,55 bis 2,2 eingestellt werden.

Außerdem ist Gegenstand der vorliegenden Erfindung die Verwendung der lagerstabilen Lösungen von carbonisiertem Magnesiumethylat in Ethanol nach Anspruch 1 zur Konservierung von Papier sowie die Verwendung der lagerstabilen Lösungen von carbonisiertem Magnesiumethylat in Ethanol nach Anspruch 1 zur Herstellung von Katalysatoren für die Polymerisierung von Olefinen.

Bei der Herstellung der erfindungsgemäßen Lösungen von carbonisiertem Magnesiumethylat dient als Magnesiumquelle entweder metallisches Magnesium (siehe Gleichung 1) oder Magnesiumethylat (siehe Gleichung 2). Die Verwendung von metallischem Magnesium ist wirtschaftlich vorteilhafter, da Magnesiumethylat üblicherweise aus Magnesiummetall durch Umsetzung mit Ethanol gewonnen wird und somit ein Reaktionsschritt entfällt. Weiterhin lassen sich bei der Umsetzung von metallischem Magnesium mit Ethanol und $CO_2$ gemäß Gleichung (1) bezüglich der Farbqualität hochwertigere Produkte erzeugen. Man erhält also bei der Umsetzung von metallischem Magnesium mit Ethanol und $CO_2$ erfindungsgemäße Lösungen, die im Vergleich mit den aus Magnesiumethylat und $CO_2$ gemäß Gleichung (2) hergestellten erfindungsgemäßen Lösungen etwas geringere Farbzahlen, gemessen nach der Meßmethode von Gardner, aufweisen.

Aufgrund der Tatsache, daß bei der Umsetzung von metallischem Magnesium mit Ethanol und $CO_2$ die Oberfläche des Magnesiums wegen der Löslichkeit des carbonisierten Magnesiumethylats ständig freiliegt, erfolgt keine Passivierung der Metalloberfläche durch die Bildung einer unlöslichen Grenzschicht zwischen Metall und Ethanol. Man ist daher bei der Herstellung der erfindungsgemäßen Lösungen gemäß Gleichung (1) nicht auf die Verwendung von oberflächenreichem Magnesiummaterial, wie z. B. Magnesiumspänen oder Magnesiumgrieß, angewiesen, wie es beispielsweise bei der technischen Herstellung von Magnesiumethylat benötigt wird, sondern kann preislich günstigere und sicherheitstechnisch unproblematischer zu handhabende Magnesiumblockware einsetzen. Weiterhin benötigt man zum Starten der Reaktion keinerlei Hilfssubstanzen, wie beispielsweise Quecksilbersalze (siehe Liebigs Annalen der Chemie 444, 236, 1925), so daß die erreichbare Produktreinheit bei Einsatz von Magnesiummetall gemäß Gleichung (1) im Vergleich mit der bei Einsatz von Magnesiumethylat gemäß Gleichung (2) etwas größer ist.

Die Carbonisierung kann durch Einleitung von gasförmigem $CO_2$ oder durch Zugabe von flüssigem oder festem $CO_2$ in das Reaktionsgemisch aus Ethanol und Magnesium gemäß Gleichung (1) bzw. aus Ethanol und Magnesiumethylat gemäß Gleichung (2) erfolgen.

Es kann also der $CO_2$-Gehalt n der erfindungsgemäßen Lösung durch Zudosieren von gasförmigem, flüssigem oder festem $CO_2$ in das Gemisch eingestellt werden.

Eine andere Variante ist, daß der $CO_2$-Gehalt n der erfindungsgemäßen Lösung durch thermisches Austreiben von $CO_2$ aus einer einen Überschuß an $CO_2$ enthaltenden Lösung eingestellt werden kann.

Die Menge des eingebrachten $CO_2$ ist einfach über Massenbilanzierung durch Gewichtszunahme kontrollierbar. Aufgrund der Hydrolyse- und Oxidationsempfindlichkeit der erfindungsgemäßen Lösungen sind sämtliche Operationen unter Ausschluß von Luft und Feuchtigkeit durchzuführen.

Lösungen von carbonisiertem Magnesiumethylat in Ethanol mit hohem $CO_2$-Gehalt n und zugleich hoher Magnesiumkonzentration neigen zu Ausfällungen bzw. Gelierung. Ein geringer $CO_2$-Gehalt n führt bei Lagerung der Lösungen innerhalb weniger Wochen zu starken Verfärbungen.

Lösungen von carbonisiertem Magnesiumethylat in Ethanol mit einem Magnesiumgehalt von 4 Gew.-% und mit einem $CO_2$-Gehalt n < 1,55 weisen im frisch bereiteten Zustand bei Verwendung von Magnesiummetall als Edukt bei der Synthese Farbzahlen von kleiner 1 (gemessen nach "Gardner") auf. Nach 6 Wochen Lagerung tritt kontinuierlich eine Farbvertiefung zu einem intensiven Orange ein.

Lösungen von carbonisiertem Magnesiumethylat in Ethanol mit einem Magnesiumgehalt von 4 Gew.-% und mit einem $CO_2$-Gehalt n > 1,55 sind farbstabil. Auch nach mehreren Monaten Lagerung in dicht verschlossenen Glasflaschen tritt keine Verfärbung ein. Die Farbzahl nach "Gardner" wird mit Werten von < 1 bestimmt. Hingegen weisen ca. 6 Wochen lang gelagerte Produkte mit einem $CO_2$-Gehalt n < 1,55 Farbzahlen nach "Gardner" von bis zu 6 auf.

Bei Lösungen von carbonisiertem Magnesiumethylat in Ethanol mit einem Magnesiumgehalt von 2,5 bis 6 Gew.-% und einem $CO_2$-Gehalt n > 1,85 treten schon nach einigen Tagen Ausfällungen auf. Lösungen von carbonisiertem Magnesiumethylat in Ethanol mit einem Magnesiumgehalt von 2,5 bis 6 Gew.-% und mit $CO_2$-Gehalten n < 1,85 sind hingegen bezüglich Ausfällungen über Monate lagerstabil.

Grenzt man also für Lösungen von carbonisiertem Magnesiumethylat in Ethanol den $CO_2$-Gehalt auf Werte von n gleich 1,55 bis 1,85 ein, so sind Magnesiumgehalte von 2,5 bis 6 Gew.-% einstellbar, ohne daß bei Lagerung der erfindungsgemäßen Lösungen signifikante Farbveränderungen oder Ausfällungen auftreten. Bei geringeren Magnesiumgehalten der erfindungsgemäßen Lösungen ist die Variationsbreite des $CO_2$-Gehaltes n größer: Bei einem Magnesiumgehalt von 1,5 bis 2,5 Gew.-% erhält man lagerstabile Lösungen für Werte des $CO_2$-Gehaltes n im Bereich von 1,55 bis 1,90. Bei Magnesiumgehalten von kleiner als 1,5 Gew.-% sind $CO_2$-Gehalte n von 1,55 bis 2,2 möglich, ohne daß während der Lagerung der erfindungsgemäßen Lösungen über lange Zeiträume Verfärbungen, Ausfällungen und Gelierungen vorkommen.

Lösungen von carbonisiertem Magnesiumethylat in Ethanol mit einem Magnesiumgehalt von 4 Gew.-% und einem $CO_2$-Gehalt n = 1,55 bis 1,85, hergestellt durch Carbonisierung von handelsüblichem Magnesiumethylat (Normalkorn, Hersteller Hüls AG) in Ethanol, weisen kurz nach ihrer Herstellung eine Farbzahl von 2 bis 3 (gemessen nach "Gardner") auf. Verwendet man zur Herstellung anstelle von Magnesiumethylat als Ausgangsmaterial Magnesiumblockware, so erhält man bei Lösungen gleicher Magnesium- und $CO_2$-Konzentrationen Farbzahlen von < 1 (gemessen nach "Gardner").

Setzt man also als Magnesiumquelle metallisches Magnesium bei der Herstellung der erfindungsgemäßen Lösungen ein, so erhält man im Hinblick auf Reinheit und Farbqualität etwas bessere Produkte als bei Einsatz von Magnesiumethylat als Ausgangsmaterial.

Die Erfindung wird durch die folgenden Beispiele näher erläutert:

## Beispiel 1

Herstellung einer lagerstabilen Lösung von carbonisiertem Magnesiumethylat aus Magnesiumethylat und $CO_2$ in Ethanol.

In einem 2 l Dreihalskolben, ausgerüstet mit Rückflußkühler, Gaseinleitungsrohr, Stickstoffüberlagerung und KPG-Flügelrührer, werden 630 g Ethanol vorgelegt und anschließend 100 g Magnesiumethylat (Hersteller: Hüls AG) unter Stickstoffbeschleierung zugesetzt. Unter Rühren wird $CO_2$ eingeleitet. Die $CO_2$-Aufnahme wird über Gewichtskontrolle kontinuierlich verfolgt. Die Temperatur im Reaktionsraum steigt kontinuierlich an. Bei Aufnahme von 66 g $CO_2$, entsprechend einem $CO_2$-Gehalt n gleich 1,72, wird die Reaktion beendet. Die Temperatur erreicht einen Maximalwert von 56 °C. Die so erzeugte Lösung ist leicht rosafarben und trüb. Der Niederschlag setzt sich innerhalb von drei Tagen vollständig ab. Durch Dekantieren oder Zentrifugieren wird die Lösung von dem Niederschlag getrennt. Die resultierende Lösung mit einem Magnesiumgehalt von 2,6 Gew.-% ist klar und weist eine Farbzahl von 2 (gemessen nach "Gardner") auf. Nach 6 Wochen Lagerung in dichtverschlossener Glasflasche tritt weder eine Verfärbung noch Ausfällung oder Gelierung auf.

## Beispiel 2

Herstellung einer lagerstabilen Lösung von carbonisiertem Magnesiumethylat aus metallischem Magnesium und $CO_2$ in Ethanol.

In einem 4 l Doppelmantel-Dreihalskolben, ausgerüstet mit Rückflußkühler, Gaseinleitungsrohr, Stickstoffüberlagerung und KPG-Flügelrührer, werden 2 374 g Ethanol vorgelegt und anschließend 121 g Magnesiumblockware (Hersteller: Normag), die auf eine Kantenlänge von 9 cm x 2 cm x 2 cm zurechtgesägt worden ist, unter Stickstoffbeschleierung in die Reaktionsapparatur mittels Edelstahldraht so eingehängt, daß sie vollständig in Ethanol eintaucht. Unter Rühren wird $CO_2$ eingeleitet. Die $CO_2$-Aufnahme wird über Gewichtskontrolle kontinuierlich verfolgt. Mittels Thermostatbeheizung wird leichter Rückfluß eingestellt. Nach Aufnahme von 378 g $CO_2$, entsprechend einem $CO_2$-Gehalt n gleich 1,7, hat sich das Magnesium unter Wasserstoffentwicklung gelöst, und die Reaktion wird nach ca. 30 h beendet. Die verbleibende Lösung ist leicht rosafarben und trüb. Der Niederschlag setzt sich innerhalb von drei Tagen vollständig ab. Durch Dekantieren oder Zentrifugieren wird die Lösung von dem Niederschlag getrennt. Die resultierende Lösung mit einem Magnesiumgehalt von 4,2 Gew.-% ist klar und weist eine Farbzahl von kleiner als 1 (gemessen nach "Gardner") auf. Nach 6 Wochen Lagerung in dichtverschlossener Glasflasche tritt weder eine Verfärbung noch Ausfällung oder Gelierung auf.

## Vergleichsbeispiel 1

Herstellung einer farblich instabilen Lösung von carbonisiertem Magnesiumethylat aus Magnesiumethylat und $CO_2$ in Ethanol.

Zunächst wird eine Lösung von carbonisiertem Magnesiumethylat in Ethanol analog zu Beispiel 1 hergestellt. Zusätzlich wird durch Einstellung eines leichten Rückflusses in ca. 3 h ein Teil des $CO_2$ durch Kochen ausge-

trieben. Der $CO_2$-Gehalt der Lösung beträgt n gleich 1,13 und der Magnesiumgehalt 2,7 Gew.-%. Nach 6 Wochen Lagerzeit ist die Lösung orangefarben, die Farbzahl steigt auf einen Wert von 5 (gemessen nach "Gardner").

Vergleichsbeispiel 2

Herstellung einer bezüglich Ausfällungen instabilen Lösung von carbonisiertem Magnesiumethylat aus Magnesiumethylat und $CO_2$ in Ethanol.
Zunächst wird eine Lösung analog zu Beispiel 1 hergestellt. Vor Abtrennung von dem Niederschlag wird die $CO_2$-Einleitung fortgesetzt. Nach zusätzlicher Aufnahme von 10 g $CO_2$, entsprechend einem jetzigen $CO_2$-Gehalt von n gleich 2,0 wird die $CO_2$-Einleitung beendet und, wie in Beispiel 1 beschrieben, von dem Niederschlag getrennt.
Die so erhaltene Lösung mit einem Magnesiumgehalt von 2,6 Gew.-% ist vollständig klar. Nach 6 Wochen Lagerzeit bei Raumtemperatur hat sich in der Lösung ein kristalliner Niederschlag gebildet. Die Niederschlagsbildung wird beschleunigt durch Lagerung bei Temperaturen unter Zimmertemperatur. Bei 10 °C fällt schon nach einigen Tagen Lagerung eine erhebliche Menge des kristallinen Niederschlages aus.

**Patentansprüche**

1. Lagerstabile Lösungen von carbonisiertem Magnesiumethylat der Formel $Mg(C_2H_5O)_2$ $(CO_2)_n$ in Ethanol,
dadurch gekennzeichnet,
daß der Magnesiumgehalt der Lösung 2,5 bis 6 Gew.-%, bezogen auf die gesamte Lösung, und der $CO_2$-Gehalt n 1,55 bis 1,85 beträgt
oder der Magnesiumgehalt der Lösung 1,5 bis 2,5 Gew.-%, bezogen auf die gesamte Lösung, und der $CO_2$-Gehalt n 1,55 bis 1,90 beträgt
oder der Magnesiumgehalt der Lösung kleiner als 1,5 Gew.-%, bezogen auf die gesamte Lösung, ist und der $CO_2$-Gehalt n 1,55 bis 2,2 beträgt.

2. Verfahren zur Herstellung lagerstabiler Lösungen von carbonisiertem Magnesiumethylat der Formel $Mg(C_2H_5O)_2$ $(CO_2)_n$ in Ethanol nach Anspruch 1 durch Umsetvon metallischem Magnesium mit Ethanol und $CO_2$,
dadurch gekennzeichnet,
daß der Magnesiumgehalt der Lösung auf Werte von 2,5 bis 6 Gew.-%, bezogen auf die gesamte Lösung, und der $CO_2$-Gehalt auf Werte von n gleich 1,55 bis 1,85
oder der Magnesiumgehalt der Lösung auf Werte von 1,5 bis 2,5 Gew.-%, bezogen auf die gesamte Lösung, und der $CO_2$-Gehalt auf Werte von n gleich 1,55 bis 1,90
oder der Magnesiumgehalt der Lösung auf Werte von kleiner als 1,5 Gew.-%, bezogen auf die gesamte Lösung, und der $CO_2$-Gehalt auf Werte von n gleich 1,55 bis 2,2 eingestellt werden.

3. Verfahren zur Herstellung lagerstabiler Lösungen von carbonisiertem Magnesiumethylat der Formel $Mg(C_2H_5O)_2$ $(CO_2)_n$ in Ethanol nach Anspruch 1 durch Umsetzung von Magnesiumethylat in Ethanol mit $CO_2$,
dadurch gekennzeichnet,
daß der Magnesiumgehalt der Lösung auf Werte von 2,5 bis 6 Gew.-%, bezogen auf die gesamte Lösung, und der $CO_2$-Gehalt auf Werte von n gleich 1,55 bis 1,85
oder der Magnesiumgehalt der Lösung auf Werte von 1,5 bis 2,5 Gew.-%, bezogen auf die gesamte Lösung, und der $CO_2$-Gehalt auf Werte von n gleich 1,55 bis 1,90
oder der Magnesiumgehalt der Lösung auf Werte von kleiner als 1,5 Gew.-%, bezogen auf die gesamte Lösung, und der $CO_2$-Gehalt auf Werte von n gleich 1,55 bis 2,2 eingestellt werden.

4. Verfahren nach den Ansprüchen 2 und 3,
dadurch gekennzeichnet,
daß der $CO_2$-Gehalt n der Lösung durch Zudosieren von gasförmigem, flüssigem oder festem $CO_2$ in das Gemisch eingestellt wird.

5. Verfahren nach den Ansprüchen 2 und 3,
dadurch gekennzeichnet,
daß der $CO_2$-Gehalt n der Lösung durch thermisches Austreiben von $CO_2$ aus einer einen Überschuß an $CO_2$ enthaltenden Lösung eingestellt wird.

6. Verwendung der lagerstabilen Lösungen von carbonisiertem Magnesiumethylat in Ethanol nach Anspruch 1 zur Konservierung von Papier.

7. Verwendung der lagerstabilen Lösungen von carbonisiertem Magnesiumethylat in Ethanol nach Anspruch 1 zur Herstellung von Katalysatoren für die Polymerisierung von Olefinen.

**Claims**

1. Storage-stable solutions of carbonated magnesium ethylate of the formula $Mg(C_2H_5O)_2$ $(CO_2)_n$ in ethanol, characterized in that the magnesium content of the solution is 2.5 to 6% by weight, relative to the total solution, and the $CO_2$ content n is 1.55 to 1.85, or the magnesium content of the solution is 1.5 to 2.5% by weight, relative to the total solution, and the $CO_2$ content n is 1.55 to 1.90, or the magnesium content of the solution is less than 1.5% by weight, relative to the total solution, and the $CO_2$ content n is 1.55 to 2.2.

2. Process for preparing storage-stable solutions of carbonated magnesium ethylate of the formula $Mg(C_2H_5O)_2 (CO_2)_n$ in ethanol according to Claim 1 by reacting metallic magnesium with ethanol and $CO_2$, characterized in that the magnesium content of the solution is adjusted to values of 2.5 to 6% by weight, relative to the total solution, and the $CO_2$ content is adjusted to values of n equal to 1.55 to 1.85, or the magnesium content of the solution is adjusted to values of 1.5 to 2.5% by weight, relative to the total solution, and the $CO_2$ content is adjusted to values of n equal to 1.55 to 1.90, or the magnesium content of the solution is adjusted to values of less than 1.5% by weight, relative to the total solution, and the $CO_2$ content is adjusted to values of n equal to 1.55 to 2.2.

3. Process for preparing storage-stable solutions of carbonated magnesium ethylate of the formula $Mg(C_2H_5O)_2 (CO_2)_n$ in ethanol according to Claim 1 by reacting magnesium ethylate in ethanol with $CO_2$, characterized in that the magnesium content or the solution is adjusted to values of 2.5 to 6% by weight, relative to the total solution, and the $CO_2$ content is adjusted to values of n equal to 1.55 to 1.85, or the magnesium content of the solution is adjusted to values of 1.5 to 2.5% by weight, relative to the total solution, and the $CO_2$ content is adjusted to values of n equal to 1.55 to 1.90, or the magnesium content of the solution is adjusted to values of less than 1.5% by weight, relative to the total solution, and the $CO_2$ content is adjusted to values of n equal to 1.55 to 2.2.

4. Process according to Claims 2 and 3, characterized in that the $CO_2$ content n of the solution is adjusted by metered addition of gaseous, liquid or solid $CO_2$ to the mixture.

5. Process according to Claims 2 and 3, characterized in that the $CO_2$ content n of the solution is adjusted by thermally expelling $CO_2$ from a solution containing an excess of $CO_2$.

6. Use of the storage-stable solutions of carbonated magnesium ethylate in ethanol according to Claim 1 for preserving paper.

7. Use of the storage-stable solutions of carbonated magnesium ethylate in ethanol according to Claim 1 for preparing catalysts for the polymerization of olefins.

## Revendications

1. Solutions stables au stockage d'éthylate de magnésium « carbonisé » de formule générale $Mg(C_2H_5O)_2 (CO_2)_n$ dans l'éthanol, caractérisée en ce que
la teneur en magnésium de la solution s'élève de 2,5 à 6 % en poids, rapporté à la solution totale et la teneur en $CO_2$ exprimée par n, de 1,55 à 1,85,
ou bien la teneur en magnésium de la solution s'élève de 1,5 à 2,5 % en poids, rapporté à la solution totale et la teneur en $CO_2$ exprimée par n de 1,55 à 1,90,
ou bien la teneur en magnésium de la solution est plus petite que 1,5 % en poids, rapporté à la solution totale et la teneur en $CO_2$ exprimée par n s'élève de 1,55 à 2,2.

2. Procédé d'obtention de solutions stables au stockage d'éthylate de magnésium carbonisé de formule $Mg(C_2H_5O)_2 (CO_2)_n$ dans l'éthanol selon la revendication 1, par mise en réaction de magnésium métallique avec de l'éthanol et du $CO_2$,
caractérisé en ce que
la teneur en magnésium de la solution est ajustée à des valeurs allant de 2,5 à 6 % en poids, rapporté à la solution totale et la teneur en $CO_2$ à des valeurs de n égales à 1,55 jusqu'à 1,85,
ou bien la teneur en magnésium de la solution est ajustée à des valeurs de 1,5 à 2,5 % en poids, rapporté à la solution totale, et la teneur en $CO_2$ à des valeurs de n égales à 1,55 jusqu'à 1,90,
ou bien la teneur en magnésium de la solution est ajustée à des valeurs inférieures à 1,5 % en poids, rapporté à la solution totale et la teneur en $CO_2$ à des valeurs de n égales à 1,55 jusqu'à 2,2.

3. Procédé d'obtention de solutions stables au stockage d'éthylate de magnésium carbonisé de formule $Mg(C_2H_5O)_2 (CO_2)n$ dans l'éthanol selon la revendication 1, par mise en réaction d'éthylate de magnésium dans l'éthanol avec $CO_2$,
caractérisé en ce que
la teneur en magnésium de la solution est ajustée à des valeurs de 2,5 à 6 % en poids, rapporté à la solution totale et la teneur en $CO_2$ à des valeurs de n égales à 1,55 jusqu'à 1,85,
ou bien la teneur en magnésium de la solution est ajustée à des valeurs de 1,5 à 2,5 % en poids, rapporté à la solution totale et la teneur en $CO_2$ à des valeurs de n égales à 1,55 jusqu'à 1,90,
ou bien la teneur en magnésium de la solution est ajustée à des valeurs inférieures à 1,5 % en poids, rapporté à la solution totale et la teneur en $CO_2$ à des valeurs de n égales à 1,55 jusqu'à 2,2.

4. Procédé selon les revendications 2 et 3,
caractérisé en ce que
la teneur en $CO_2$ n de la solution est ajustée par addition dosée de $CO_2$ gazeux, liquide ou solide dans le mélange.

5. Procédé selon les revendications 2 et 3,
caractérisé en ce que
la teneur en $CO_2$ n de la solution est ajustée par élimination par la chaleur de $CO_2$ d'une solution contenant un excès de $CO_2$,

6. Utilisation de solutions stables au stockage d'éthylate de sodium carbonisé dans l'éthanol selon la revendication 1, pour la conservation du papier.

7. Utilisation de solutions stables au stockage d'éthylate de magnésium carbonisé dans l'éthanol selon la revendication 1, pour la préparation de catalyseurs pour la polymérisation d'oléfines.